(19) Europäisches Patentamt
European Patent Office
Office européen des brevets

(11) Veröffentlichungsnummer: **0 492 188 A2**

(12) # EUROPÄISCHE PATENTANMELDUNG

(21) Anmeldenummer: **91120769.4**

(22) Anmeldetag: **03.12.91**

(51) Int. Cl.[5]: **C08F 8/00**, C02F 1/56

(30) Priorität: **28.12.90 SU 4891956**

(43) Veröffentlichungstag der Anmeldung:
**01.07.92 Patentblatt 92/27**

(84) Benannte Vertragsstaaten:
**AT BE CH DE DK ES FR GB GR IT LI LU NL SE**

(71) Anmelder: **OPTIMATION Industrieberatung für Laser- und analytische Messtechnik GmbH**
**Rottmannstrasse 11a**
**W-8000 München 2(DE)**

(72) Erfinder: **Kasaikin, Viktor A.**
**ul. Uralskaja 6/4/96**
**SU-107 207 Moskau(SU)**
Erfinder: **Zezin, Alexander B.**
**ul. Novokuznetkaya 17/19/137**
**SU-113 184 Moskau(SU)**
Erfinder: **Kabanov, Viktor A.**
**Lomonosovksy pros.14/108**
**SU-117 296 Moskau(SU)**
Erfinder: **Kuzjakov, Jakob J.**
**ul. Novoorlovskaja 8/2/139**
**SU-119 633 Moskau(SU)**
Erfinder: **Borodulina, Tatjana A.**
**ul. Klimova 44b/67**
**SU-142 412 Noginsk, Moskau Bezirk(SU)**
Erfinder: **Bronitsch, Tatjana K.**
**ul. 1. Novokusminskaja 23/1/24**
**SU-109 377 Moskau(SU)**
Erfinder: **Sergeev, Vladimir G.**
**Solovinii projezd 4/1/134**
**SU-117 593 Moskau(SU)**
Erfinder: **Krinner, Rudolf**
**Rottmannstrasse 11A**
**W-8000 München(DE)**

(74) Vertreter: **Reinhard, Skuhra, Weise**
**Friedrichstrasse 31**
**W-8000 München 40(DE)**

(54) **Polyelektrolytkomplexe.**

(57) Es wird ein neuer Komplex eines Polyelektrolyten mit einem gegensätzlich geladenen oberflächenaktiven Mittel, ein Verfahren zur Herstellung dieses Komplexes und die Verwendung dieses Komplexes zur Reinigung von Abwässern beschrieben.

EP 0 492 188 A2

Die Erfindung betrifft neue Polyelektrolytkomplexe, ein Verfahren zur Herstellung dieser Komplexe und ihre Verwendung.

Es ist bekannt, Abwässer zur Reinigung von organischen Verbindungen mit Sorbentien zu behandeln. Als Sorbentien werden vernetzte (Co-) Polymere verwendet, z.B. Sterolcopolymere mit Divinylbenzol (Polysorb 40/100) (Copyright Certificate No. 1331832, USSR, cl. C 02 F/28, 1987. Copyright Certificate No. 1143694, USSR, cl. C 02 F 1/28 1985). Diese Copolymere zeigen jedoch nur eine geringe Sorbtionskapazität und ihre Fähigkeit, Abwässer von hydrophoben aromatischen Verbindungen (z.B. Phenol) und aliphatischen Kohlenwasserstoffen zu reinigen, ist gering.

Weiterhin ist bekannt, Polvvinylbenzyl-N-trimethylammoniumchlorid als Flockungsmittel bei der Abwasserreinigung einzusetzen (Copyright Certificate No. 829582, USSR, cl. C 02 F 1/56, 1979). Dieses Flockungsmittel ist jedoch eine wasserlösliche Verbindung, die nur in Gegenwart von Metalloxiddispersionen wirksam ist.

Weiterhin ist bekannt, organische Verbindungen enthaltende Abwässer zur Aufreinigung mit Flockungsmitteln zu behandeln, die auf einem Polyelektrolytkomplex basieren, so z.B. nichtstöchiometrische Komplexe aus Polyacrylamid mit Alkyltrimethylammoniumsalz ((Copyright Certificate No. 1346585, USSR, cl. C 02 F 1/56, 1984). Als Alkyltrimethylammoniumsalz werden Dodecyltrimethylammoniumbromid, Tetradecyltrimethylammoniumbromid, Cetyltrimethylammoniumbromid oder Octadecyltrimethylammoniumbromid verwendet. Das Flockungsmittel wird in einer Menge von 0,2 - 0,8 mg/l zugegeben. Dieser Polykomplex ist ein wasserlösliches Polymer. Aus diesem Grund kann dieser Polykomplex auch nur als Flockungsmittel von mineralischen und organischen Dispersionen verwendet werden, dagegen nicht zur Reinigung von Wasser von löslichen organischen Beimengungen.

Es ist eine Aufgabe der Erfindung, neue Polyelektrolytkomplexe sowie ein Verfahren zu ihrer Herstellung bereitzustellen.

Es ist eine weitere Aufgabe der Erfindung, den Grad der Wasserreinigung, insbesondere von organischen Verbindungen aus Abwässern, zu verbessern, und die Nachteile des Standes der Technik zu vermeiden.

Der neue Komplex ist gekennzeichnet durch einen Komplex eines Polyelektrolyten (PE) mit einem gegensätzlich geladenen oberflächenaktiven Mittel (SAA), enthaltend a) 50 - 100 Mol-% an Monomereinheiten der folgenden allgemeinen Formel:

[-X-]•[R-Y],

wobei [-X-] die PE-funktionelle Gruppe darstellt und X ausgewählt ist aus einer oder mehreren der folgenden anionischen PE-funktionellen Gruppen mit

X= 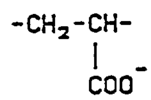 , 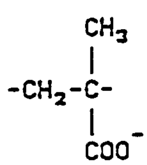 , 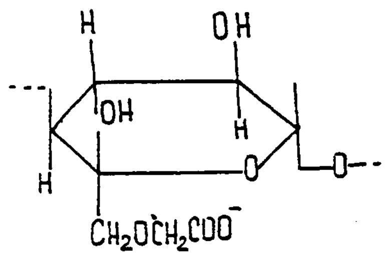 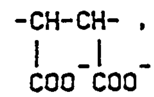 ,

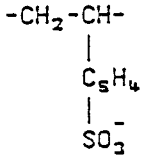 , 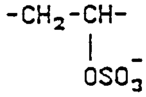 , 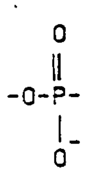 ,

und/oder aus einer oder mehreren der folgenden kationischen PE-funktionellen Gruppen mit

X =

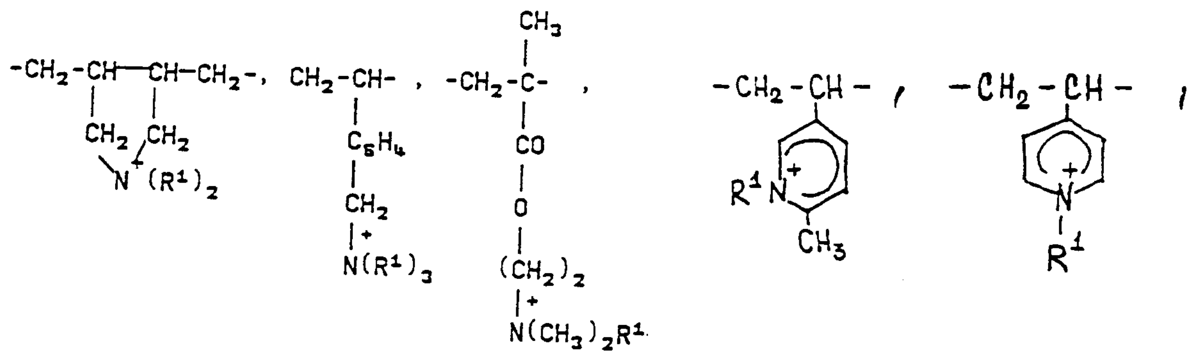

$-CH_2-CH_2-N^+H(R^3)-$ ,

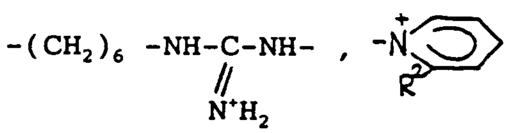

und

[R-Y] die SAA-funktionelle Gruppe darstellt, wobei Y ausgewählt ist aus einer oder mehreren der folgenden Gruppen mit

Y = $-N^+(R^2)_2R^4$ , $-OSO^-_3$ , $-SO^-_3$ , $-COO^-$ ,

und wobei R und $R^1$ bis $R^4$ folgende Bedeutungen aufweisen:

R = $C_nH_{2n+1}$ , $C_nH_{2n-1}$ , $C_{20}H_{37}O_4$ , n >10 ,

$R^1$ = H, $CH_3$ , $C_2H_5$ , $C_3H_7$,

$R^2$ = H, $CH_3$ ,

$R^3$ = H, $-[(CH_2-CH_2-NH)-]_x-CH_2-CH_2-NH_2$ , x = O,1,2

$R^4$ = H, $CH_3$ , $C_2H_5$

und

b) wahlweise 0 - 50 Mol.% an nicht-ionogenen Monomereinheiten.

Für n in der Formel R = $C_nH_{2n+1}$, $C_nH_{2n-1}$ gibt es prinzipiell keine festgelegte Obergrenze. Bevorzugt ist aber ein Bereich von 10<n<22.

Der erfindungsgemäße Komplex ist unlöslich in Wasser und elektrisch neutral.

Als Polyelektrolyten sind sowohl anionische als auch kationische Polyelektrolyten (PE) verwendbar. Die anionischen Polyelektrolyten werden bevorzugt ausgewählt aus einem der folgenden Polyelektrolyten oder deren Mischungen sowie ihren Salzen:

Carboxylgruppen enthaltenden anionischen Polyelektrolyten, insbesondere Polyacrylsäure (PAA), Polymethacyrlsäure (PMAA), hydrolysiertes Poly(acrylnitril) (HYPAN), Styromal (alternierendes Styrol- und Maleinsäurecopolymer), oxidiertes Lignin, Carboxymethylzellulose;

Sulfat- und Sulfo-Gruppen enthaltenden anionischen Polyelektrolyten, insbesondere Polystyrolsulfonsäure (PSSA) und Polyvinylsulfat;

Phosphatgruppen enthaltenden anionischen Polyelektrolyten, insbesondere Natriumpolyphosphat.

Die kationischen Polyelektrolyten werden bevorzugt ausgewählt aus einem der folgenden Polyelektrolyten oder deren Mischungen sowie ihren Salzen:

Aliphatische Amin- und Ammoniumgruppen enthaltenden kationischen Polyelektrolyten, insbesondere Polydimethyldiallylammoniumchlorid (PDMDAAC), Polyethylenimin (PEI), Poly-N,N-Dimethylaminoethylmethacrylat (PDMAEMA), Poly-p-Vinylbenzyltrimethylammoniumchlorid (PVBAC);

aromatische Amin- und Ammoniumgruppen enthaltenden kationischen Polyelektrolyten, insbesondere Poly-4-Vinyl-N-alkylpyridiniumhalogenid (QPVP) und Poly-N-Ethyl-2-methyl-5-vinyl-pyridinium-halogenid;

Guanidino-Gruppen-enthaltenden kationischen Polyelektrolyten, insbesondere Polyhexamethylen-guanidino-halogenid.

Als Polyelektrolyten sind erfindungsgemäß verwendbar Homo- und/oder Copolymere natürlicher oder synthetischer Herkunft. Das Copolymer kann bevorzugt nicht-ionogene Einheiten enthalten, bevorzugt Einheiten vom Vinyl-, Dien-, Allyl-, Glucopyranose- oder Coniferyl-Typ, insbesondere bevorzugt mit (Meth)-acrylamid, Styrol, Alkyl(meth)acrylat, Vinylpyrrolidon, Vinylacetat, Glucopyranose, Coniferylalkohol.

Die Eigenschaften der bevorzugt verwendbaren Polyelektrolyten sind in der untenstehenden Tabelle 1 angegeben.

Tabelle 1

| Eigenschaften der Polyelektrolyten (PE) | | |
|---|---|---|
| PE | $M_w x10^{-3}$ | USSR-Standard oder Herstellerfirma |
| PAC | 40-300 | Aldrich-Chemie |
| PMAC | 19-400 | Aldrich-Chemie |
| PSSA | 200 | Aldrich-Chemie |
| hydrolysiertes PAN | | TY 6-OI-I66-74 |
| PDMDAAC | | TY 6-05-2009-86 |
| PEI | 14-20 | Polyimin |
| PDMAEMA | 100-500 | Serva |
| QPVP | 70-300 | Aldrich-Chemie |
| PVBAC | 100-500 | Serva |

Als oberflächenaktive Mittel (SAA) können kationische und/oder anionische oberflächenaktive Mittel verwendet werden. Die anionisch oberflächenaktiven Mittel werden bevorzugt ausgewählt aus einem oder mehreren der folgenden oberflächenaktiven Mitteln: Sulfat-Gruppen-enthaltenden anionischen oberflächenaktiven Mitteln, insbesondere Dodecylsulfat (DSMe), Cetylsulfat (CSMe); Sulfonat-Gruppen-enthaltenden anionischen oberflächenaktiven Mitteln, insbesondere Me-Diisooctylsulfosuccinat, Me-decylsulfonat, Tetradecylsulfonat; Carboxylat-Gruppen-enthaltenden anionischen oberflächenaktiven Mitteln, insbesondere Salzen der Fettsäuren, bevorzugt Me-oleat, Me-palmitat, wobei je Me = $Li^+$, $Na^+$, $K^+$.

Die kationisch oberflächenaktiven Mittel werden bevorzugt ausgewählt aus einem oder mehreren der folgenden kationischen oberflächenaktiven Mittel: Amin- oder Ammoniumgruppen-enthaltenden kationischen oberflächenaktiven Mitteln, insbesondere Dodecyltrimethylammoniumbromid (DTMAB), Tetradecyltrimethylammoniumbromid (TDTMAB), Cetylmethylammoniumbromid (CTMAB), Cetylpyridiniumchlorid (CPC)-;Pyridiniumgruppen-enthaltenden kationischen oberflächenaktiven Mitteln, insbesondere Dodecylpydridinium-halogenid, Tetradecyl-pyridinium-halogenid, Cetylpyridiniumchlorid (CPC).

Die SAA-Moleküle sollten erfindungsgemäß bevorzugt nicht weniger als einen aliphatischen oder alkylaromatischen Rest enthalten, wobei die Zahl der Kohlenstoffatome im Bereich von 1 bis 20, bevorzugt im Bereich von 12 bis 16, liegt.

Als ionogene Gruppen in den PE-Makromolekülen und SAA-Molekülen können bevorzugt Carboxyl-, Carboxylat-, Sulfat-, Phosphat-, Amin-, Ammonium-, Pyridin- oder Guaningruppen verwendet werden.

Bevorzugt enthält der Komplex 60 - 100 Mol-%, 70 - 90 Mol-%, 75 - 85 Mol.% oder 100 Mol-% an Monomereinheiten der allgemeinen Formel [-X-]•[R-Y] und 0 - 40 Mol-%, 10 - 30 Mol-%, 15 - 25 Mol-% oder 0 Mol-% an nicht-ionogenen Monomereinheiten.

Das molare Verhältnis PE : SAA liegt im Bereich von 1:1 bis 0,5, bevorzugt im Bereich von 1:1 bis 0,7.

Das molare Verhältnis PE : SAA liegt bevorzugt im Bereich von 1:1.

Der Polymerisationsgrad des Komplexes der allgemeinen Formel $([-X-]\bullet[R-Y])_m$ liegt bevorzugt im Bereich von m = 200-4650, weiterhin bevorzugt im Bereich von 220-4500.

Die erfindungsgemäßen Komplexe werden bevorzugt dadurch hergestellt, daß das PE und SAA in einem üblichen Lösungsmittel vermischt werden und das entstehende Präzipitat aus der flüssigen Phase durch an sich bekannte Techniken zur Phasentrennung bei Suspensionen entfernt wird. Weiterhin werden PE und SAA in einem üblichen Lösungsmittel, bevorzugt Wasser bei einer Temperatur im Bereich von 5 °C - 100 °C in einem Verhältnis der ionogenen Gruppen PE : SAA von 1 : 1 - 0,5 vermischt und das entstehende Präzipitat wird aus der flüssigen Phase durch an sich bekannte Techniken zur Phasentrennung bei Suspensionen entfernt.

Die erfindungsgemäßen Komplexe sind weiterhin durch Polymerisierung oder Copolymerisierung von Monomeren (Comonomeren) in SAA-Lösungen herstellbar. Bei dieser Copolymerisation entsteht ein PE-SAA-Komplex, welcher präzipitiert. Der PE-SAA-Komplex ist eine chemische Verbindung, die durch das Vermischen wäßriger Lösungsmittel mit den gegensätzlich geladenen Polyelektrolyten und oberflächenaktiven Mitteln entsteht. Der Komplex wird, wie oben angegeben, durch an sich bekannte Techniken, wie z.B. Filtration oder Präzipitation, entfernt.

Die erfindungsgemäß bereitgestellten Komplexe wirken als Sorbens und sind insbesondere zur Reinigung von Abwässern von organischen Verbindungen, insbesondere von hydrophoben aromatischen Verbindungen und aliphatischen Kohlenwasserstoffen, geeignet.

Eine weitere Aufgabe der Erfindung ist es, ein Verfahren zur Abwasserreinigung von organischen Molekülen bereitzustellen, welches effektiver arbeitet als die Verfahren nach dem Stand der Technik.

Das erfindungsgemäße Verfahren ist dadurch gekennzeichnet, daß eine Suspension mit den erfindungsgemäßen Polyelektrolytkomplexen, welche die organischen Fremdmoleküle enthält, in Wasser hergestellt wird, diese Suspension wenige Minuten (im Durchschnitt 20 Minuten) gerührt und anschließend die flüssige Phase von der festen Phase durch Präzipitation und/oder Filtration entfernt wird. Erfindungsgemäß sind auch Säulentechniken verwendbar.

Die Effektivität der erfindungsgemäß bereitgestellten Sorbentien beruht wahrscheinlich auf der Tatsache, daß in den PE-Komplexen die SAA-Moleküle intramolekulare Micellen bilden, in welchen sie in amorphem und nicht im kristallinen Zustand vorliegen. Dies wurde durch Röntgenstrukturanalyse und Differential-Scanning-Kalorimetermessung festgestellt. Im SAA können die organischen Moleküle gelöst werden, während der Komplex selbst in dem Wasser unlöslich bleibt.

Bevorzugt liegt das Verhältnis Polyelektrolytkomplex : Wasser im Bereich von 1 : 10 - 1 : 100

## Beispiele zur Herstellung der erfindungsgemäßen Komplexe

### Beispiel 1

Herstellung eines Natriumpolymethacrylat- und Dodecyltrimethylammoniumbromidkomplexes.

Eine Lösung aus 0,005 g Natriumpolymethacrylat in 1 ml Wasser wird mit einer Lösung aus 0,014 g Dodecyltrimethylammoniumbromid in 1 ml Wasser bei 20 °C vermischt. Der präzipitierte Komplex wird dekantiert und getrocknet. Es werden 0,17 g Sorbens erhalten. Die theoretische Ausbeute beträgt 89,5%.

### Beispiel 2

Herstellung eines Polymethacrylsäure- und Cetyltrimethylammoniumbromid-Komplexes durch Polymerisierung

In 10 ml Wasser werden nach und nach 0,364 g Cetyltrimethylammoniumbromid, 0,172 g Methacrylsäure, 0,08 g NaOH und 3 x $10^{-4}$ g Kalium gelöst. Die erhaltene Lösung wird in ein Glasgefäß überführt und durch dieses Glasgefäß 30 Minuten lang Stickstoff geleitet. Anschließend wird das Glasgefäß verschmolzen und in einen Thermostaten bei 80 °C überführt. Nach 36 Stunden wird das Glasgefäß abgekühlt, geöffnet, das Präzipitat in einem Buchner-Trichter filtriert und anschließend bis zur Erreichung eines konstanten Gewichtes getrocknet. Es werden 0,294 g des Sorbens erhalten. Die theoretische Ausbeute beträgt 62 %.

### Beispiel 3

Herstellung eines Polyacrylat- und Tetradecyltrimethylammoniumbromid-Komplexes

Eine Lösung aus 0,03 g Natriumpolyacrylat in 4 ml Wasser wird mit einer Lösung aus 0,93 g Tetradecyltrimethylammoniumbromid in 5 ml Wasser bei 40 °C vermischt. Der als Bodenstand vorliegende Komplex wird durch Dekantierung abgetrennt. Es werden 0,12 g Sorbens erhalten. Die theoretische Ausbeute beträgt 85,7 %.

### Beispiel 4

Herstellung eines Komplexes aus Hydrochlorid-Poly-N,N-dimethylaminoethylmethacrylat und Natriumdodecylsulfat

Eine Lösung aus 0,45 g Poly-N,N-Dimethylaminoethylmethacrylathydrochlorid wird mit einer Lösung aus 0,67 g Natriumdodecylsulfat in 5 ml Wasser vermischt. Der in Form eines Bodenstandes vorliegende Komplex wird durch Dekantierung abgetrennt und getrocknet. Es werden 1,05 g Sorbens erhalten. Die theoretische Ausbeute beträgt 93,8 %.

Beispiel 5

Herstellung eines Komplexes aus Natriumpolyacrylat und Cetyltrimethylammoniumbromid

Eine Lösung aus 0,03 g Natriumpolyacrylat in 4 ml Wasser wird mit einer Lösung aus 1,01 g Cetyltrimethylammoniumbromid in 8 ml Wasser bei 60° C vermischt. Der in Form eines Bodensatzes vorliegende Komplex wird durch Dekantierung getrennt und getrocknet. Es werden 0,125 g Sorbens erhalten. Die theoretische Ausbeute beträgt 96,2 %.

Beispiele zur Bestimmung der Sorptionskapazität der erfindungsgemäßen Komplexe :

Beispiel 6

Zu 10 ml einer gesättigten Benzollösung in Wasser (Konzentration 0,08 %) werden 0,5 g des Sorbens aus Beispiel 1 gegeben und 20 Minuten lang vermischt. Das Sorbens wird vom Wasser in einem Buchner-Trichter abgetrennt; der Restgehalt des Benzols in Wasser betrug 0,005 %, bestimmt durch Gaschromatographie oder UV-Spektroskopie. Die gleiche Menge an Sorbens wird verwendet, um die weiteren Teile an gesättigter Benzollösung in Wasser weiterzuverarbeiten, bis der Sorptionsprozeß abbricht. Durch dieses Verfahren wird die größtmögliche Sorptionskapazität des Sorbens (im Verhältnis zu Benzol) bestimmt. Sie beträgt 12,8 mmol/g.

Beispiel 7

Wie im Beispiel 6 wird das Sorbens aus Beispiel 1 zur Reinigung einer gesättigten Lösung von Toluol in Wasser (0,057 %) verwendet. Die Restkonzentration an Toluol in Wasser beträgt nach der ersten Behandlung 0,005 %, die höchste Sorptionskapazität beträgt 10,9 mmol/g.

Beispiel 8

Wie im Beispiel 6 wird das Sorbens aus Beispiel 1 zur Reinigung einer gesättigten Lösung von Styrol in Wasser (0,03 %) verwendet. Die Restkonzentration an Styrol in Wasser beträgt nach der ersten Behandlung 0,003 %, die höchste Sorptionskapazität beträgt 19,2 mmol/g.

Beispiel 9

Wie im Beispiel 7 wird das Sorbens aus Beispiel 2 zur Reinigung einer gesättigten Phenollösung in Wasser (6,7 % bei 16° C) verwendet. Die Restkonzentration an Phenol beträgt nach der ersten Behandlung 4,5 %, die maximale Sorptionskapazität beträgt 213 mmol/g.

Beispiel 10

Wie im Beispiel 7 wird die maximale Sorptionskapazität des Sorbens aus Beispiel 2 für Anthracen bestimmt. Sie beträgt 5,6 mmol/g.

Beispiel 11

Wie im Beispiel 7 wird die maximale Sorptionskapazität des Sorbens aus Beispiel 3 für Benzol bestimmt. Sie beträgt 10,7 mmol/g.

Beispiel 12

Wie im Beispiel 7 wird die maximale Sorptionskapazität des Sorbens aus Beispiel 4 für Toluol bestimmt. Sie beträgt 3,6 mmol/g.

Beispiel 13

Wie im Beispiel 7 wird die maximale Sorptionskapazität des Sorbens aus Beispiel 7 für Phenol bestimmt. Sie beträgt 13,5 mmol/g.

Beispiel 14

Die Herstellung eines Komplexes auf der Basis eines Copolymeres mit nicht-ionogenen Gruppen erfolgt beispielsweise wie folgt:
Eine Lösung aus 0,05 g hydrolysiertem Polyacrylamid (HYPAN), enthaltend 50 Mol.% Acrylateinheiten und 50 Mol.% Acrylamideinheiten in 4 ml Wasser, wird mit einer Lösung von 0,105 g Tetradecyltrimethylammoniumbromid in 5 ml Wasser bei 20° C vermischt. Der Komplex wird durch Filtration abgetrennt. Es werden 0,1 g an Sorbens erhalten. Die theoretische Ausbeute beträgt 80%.

In Tabelle 2, Nr. 4, sind die Ergebnisse einer Wasserreinigung durch ein Sorbens auf der Basis eines hydrolysierten Polyacrylamids angegeben.

In der folgenden Tabelle 2 werden weitere Beispiele für die Anwendung der erfindungsgemäßen Polyelektrolyt-SAA-Komplexe als Sorbentien zur Abtrennung von in Wasser schwach löslichen organischen Verbindungen gezeigt.

Das Beispiel Nr. 9 der Tabelle 2 zeigt ein Vergleichsbeispiel gemäß dem Stand der Technik mit Polyacrylamid als Polyelektrolyten, wie es in der Beschreibungseinleitung als Stand der Technik angeführt wurde. Der Wasserreinigungsgrad beträgt hier 0 %.

Tabelle 2

| No. | PE | SAA | Sorbat | Verhältnis Sorbens/Wasser (g/g) | Sorbens-Sorptions-Kapazität (mmol/g) | Wasser Reinigungsgrad (%) |
|---|---|---|---|---|---|---|
| I. | PMAC | DTMAB | Benzol | 1/5 | 12,8 | 100 |
| | | TDTMAB | Toluol | 1/10 | 14,9 | 98,4 |
| | | CTMAB | Styrol | 1/50 | 14,8 | 90,0 |
| | | CPC | Phenol | 1/100 | 1,7 | 52,3 |
| 2. | PAA | DTMAB | Benzopyren | 1/100 | -- | 100 |
| | | TDTMAB | Fluorescein | 1/25 | -- | 100 |
| | | CTMAB | Phenol | 1/10 | 1,7 | 71,9 |
| | | CPC | Benzol | 1/50 | 17,6 | 92,4 |
| 3. | PSAA | DTMAB | Toluol | 1/120 | 10,9 | 79,1 |
| | | TDTMAB | Anthracin | 1/50 | -- | 100 |
| | | CTMAB | Pyren | 1/5 | 7,3 | 100 |
| | | CPC | Hexan | 1/10 | -- | 80 |
| 4. | hydrol. PAN | DTMAB | Styrol | 1/50 | 5,8 | 87,6 |
| | | TDTMAB | Benzol | 1/100 | 15,4 | 85,2 |
| | | CTMAB | Anthracin | 1/100 | -- | 100 |
| | | CPC | Benzopyren | 1/5 | -- | 100 |
| 5. | PDM-DAAC | DSNa | Phenol | 1/5 | 6,8 | 91,0 |
| | | CSNa | Benzol | 1/10 | 13,8 | 85,2 |
| 6. | PDMA EMA | DSNa | Phenol | 1/5 | 8,5 | 94,3 |
| | | CSNa | Phenol | 1/100 | 15,4 | 87,6 |
| 7. | QPVP | DSNa | Anthracin | 1/100 | 5,3 | 100 |
| | | CSNa | Fluorescein | 1/75 | --- | 100 |
| 8. | PVBAC | DSNa | Styrol | 1/10 | 3,9 | 94,1 |
| | | CSNa | Anthracin | 1/20 | 8,9 | 100 |
| 9. | Polyacryl amid | DTMAB | Benzol | 1/100 | --- | 0 |
| | | TDTMAB | Styrol | 1/10 | --- | 0 |
| | | CTMAB | Toluol | 1/25 | --- | 0 |

## Patentansprüche

1. Komplex eines Polyelektrolyten (PE) mit einem gegensätzlich geladenen oberflächenaktiven Mittel (SAA) sowie dessen Salze, enthaltend

   a) 50 - 100 Mol-% an Monomereinheiten der folgenden allgemeinen Formel:

   [-X-]•[R-Y],

   wobei [-X-] die PE-funktionelle Gruppe darstellt und X ausgewählt ist aus einer oder mehreren der folgenden anionischen PE-funktionellen Gruppen mit

X= 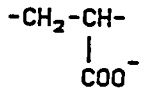 , 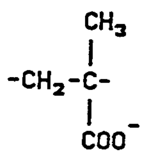 , 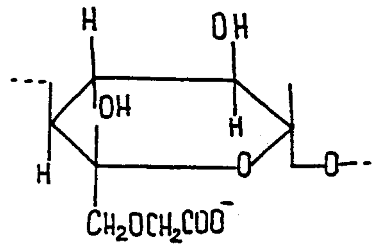 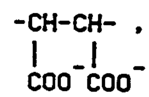 ,

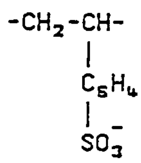 , 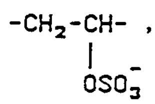 , 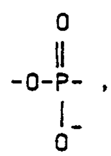 ,

   und/oder aus einer oder mehreren der folgenden kationischen PE-funktionellen Gruppen mit

X =

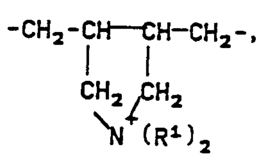 , 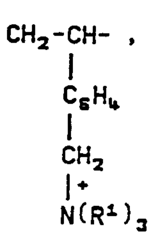 , 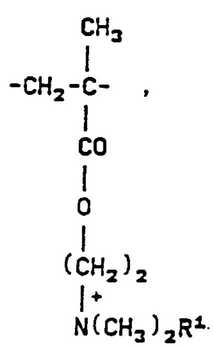 , 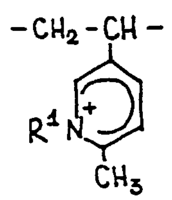 , 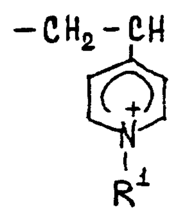 ,

$-CH_2-CH_2-N^+H(R^3)-$ , 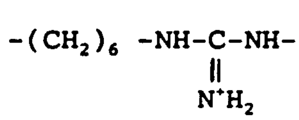 ,

und

[R-Y] die SAA-funktionelle Gruppe darstellt, wobei Y ausgewählt ist aus einer oder mehreren der folgenden Gruppen mit

Y = $-N^{+}(R^2)_2R^4$ , $-OSO^{-}_3$ , $-SO^{-}_3$ , $-COO^{-}$ ,

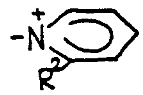

und wobei R und $R^1$ bis $R^4$ folgende Bedeutungen aufweisen:

R = $C_nH_{2n+1}$ , $C_nH_{2n-1}$ , $C_{20}H_{37}O_4$ , n >10 ,
$R^1$ = H, $CH_3$ , $C_2H_5$ , $C_3H_7$,
$R^2$ = H, $CH_3$ ,
$R^3$ = H, $-[(CH_2-CH_2-NH)-]_x-CH_2-CH_2-NH_2$ , x = O,1,2
$R^4$ = H, $CH_3$ , $C_2H_5$

und

b) wahlweise 0 - 50 Mol.% an nicht-ionogenen Monomereinheiten.

**2.** Komplex nach Anspruch 1,
dadurch gekennzeichnet,
daß der Polyelektrolyt ein anionischer und/oder kationischer Polyelektrolyt ist,
wobei vorzugsweise
der anionische Polyelektrolyt ausgewählt ist aus einem der folgenden Polyelektrolyten oder deren Mischungen sowie ihren Salzen:
Carboxylgruppen enthaltenden anionischen Polyelektrolyten, insbesondere Polyacrylsäure (PAA), Polymethacyrlsäure (PMAA), hydrolysiertes Poly(acrylnitril) (HYPAN), Styromal (alternierendes Styrol- und Maleinsäurecopolymer), oxidiertes Lignin, Carboxymethylzellulose;
Sulfat- und Sulfo-Gruppen enthaltenden anionischen Polyelektrolyten, insbesondere Polystyrolsulfonsäure (PSSA) und Polyvinylsulfat;
Phosphatgruppen enthaltenden anionischen Polyelektrolyten, insbesondere Natriumpolyphosphat,
und wobei vorzugsweise
der kationische Polyelektrolyt ausgewählt ist aus einem der folgenden Polyelektrolyten oder deren Mischungen sowie ihren Salzen:

aliphatischen Amin- und Ammoniumgruppen enthaltenden kationischen Polyelektrolyten, insbesondere Polydimethyldiallylammoniumchlorid (PDMDAAC), Polyethylenimin (PEI), Poly-N,N-Dimethylaminoethylmethacrylat (PDMAEMA), Poly-p-Vinylbenzyltrimethylammoniumchlorid (PVBAC);
aromatische Amin- und Ammoniumgruppen enthaltenden kationischen Polyelektrolyten, insbesondere Poly-4-Vinyl-N-alkylpyridiniumhalogenid (QPVP) und Poly-N-Ethyl-2-methyl-5-vinyl-pyridinium-halogenid;
Guanidino-Gruppen-enthaltenden kationischen Polyelektrolyten, insbesondere Polyhexamethylenguanidino-halogenid.

**3.** Komplex nach einem oder mehreren der vorhergehenden Ansprüche,
dadurch gekennzeichnet,
daß das oberflächenaktive Mittel (SAA) ein kationisches und/oder anionisches oberflächenaktives Mittel ist,
wobei vorzugsweise
das anionische oberflächenaktive Mittel ausgewählt ist aus einem oder mehreren der folgenden oberflächenaktiven Mitteln:
Sulfat-Gruppen-enthaltenden anionischen oberflächenaktiven Mitteln, insbesondere Dodecylsulfat (DSMe), Cetylsulfat (CSMe);
Sulfonat-Gruppen-enthaltenden anionischen oberflächenaktiven Mitteln, insbesondere Me-Diisooctylsulfosuccinat, Me-decylsulfonat, Tetradecylsulfonat;
Carboxylat-Gruppen-enthaltenden anionischen oberflächenaktiven Mitteln, insbesondere Salzen der Fettsäuren, bevorzugt Me-oleat, Me-palmitat,

wobei je Me = $Li^+$, $Na^+$, $K^+$,
und wobei vorzugsweise
das kationische oberflächenaktive Mittel ausgewählt ist aus einem oder mehreren der folgenden kationischen oberflächenaktiven Mittel:
Amin- oder Ammoniumgruppen-enthaltenden kationischen oberflächenaktiven Mitteln, insbesondere Dodecyltrimethylammoniumbromid (DTMAB), Tetradecyltrimethylammoniumbromid (TDTMAB), Cetylmethylammoniumbromid (CTMAB), Cetylpyridiniumchlorid (CPC);
Pyridiniumgruppen-enthaltenden kationischen oberflächenaktiven Mitteln, insbesondere Dodecylpydridinium-halogenid, Tetradecyl-pyridinium-halogenid, Cetylpyridiniumchlorid (CPC).

**4.** Komplex nach einem oder mehreren der vorhergehenden Ansprüche,
dadurch gekennzeichnet,
daß die Polyelektrolyten Homo- und/oder Copolymere natürlicher oder synthetischer Herkunft sind,
wobei vorzugsweise
das Homo- und/oder Copolymer nicht-ionogene Monomereinheiten enthält, bevorzugt Einheiten vom Vinyl-, Dien-, Allyl-, Glucopyranose- oder Coniferyl-Typ, insbesondere bevorzugt (Meth)acrylamid, Styrol, Alkyl(meth)acrylat, Vinylpyrrolidon, Vinylacetat, Glucopyranose, Coniferylalkohol.

**5.** Komplex nach einem oder mehreren der vorhergehenden Ansprüche,
dadurch gekennzeichnet,
daß der Komplex 60 - 100 Mol-%, 70 - 90 Mol-%, 75 - 85 Mol.% oder 100 Mol-% an Monomereinheiten der allgemeinen Formel [-X-]•[R-Y] und 0 - 40 Mol-%, 10 - 30 Mol-%, 15 - 25 Mol-% oder 0 Mol-% an nicht-ionogenen Monomereinheiten enthält.

**6.** Komplex nach einem oder mehreren der vorhergehenden Ansprüche,
dadurch gekennzeichnet,
daß das molare Verhältnis PE:SAA im Bereich von 1 : 1 - 0,5, bevorzugt im Bereich von 1:1 - 0,7, liegt, insbesondere 1:1 ist.

**7.** Komplex nach einem oder mehreren der vorhergehenden Ansprüche,
dadurch gekennzeichnet,
daß das molare Verhältnis der ionisierten ionogenen Gruppen des Polyelektrolyten und des SAA in einem Komplex 1:1 beträgt.

**8.** Komplex nach einem oder mehreren der vorhergehenden Ansprüche,
dadurch gekennzeichnet,
daß der Polymerisationsgrad des Komplexes der allgemeinen Formel $([-X-]\bullet[R-Y])_m$ bevorzugt im Bereich von m = 200-4650, insbesondere im Bereich von 220-4500 liegt.

**9.** Verfahren zur Herstellung des Komplexes nach einem oder mehreren der vorhergehenden Ansprüche,
**dadurch gekennzeichnet**,
daß PE und SAA in einem üblichen Lösungsmittel vermischt werden und das entstehende Präzipitat aus der flüssigen Phase durch an sich bekannte Techniken zur Phasentrennung bei Suspensionen entfernt wird,
wobei vorzugsweise
PE und SAA in einem üblichen Lösungsmittel, bevorzugt Wasser bei einer Temperatur im Bereich von 5 °C - 100 °C in einem Verhältnis der ionogenen Gruppen PE : SAA von 1 : 1 - 0,5 vermischt werden und das entstehende Präzipitat aus der flüssigen Phase durch an sich bekannte Techniken zur Phasentrennung bei Suspensionen entfernt wird.

**10.** Verfahren zur Herstellung des Komplexes nach einem oder mehreren der vorhergehenden Ansprüche,
dadurch gekennzeichnet,
daß die PE-Monomeren in den SAA-Lösungen polymerisiert oder copolymerisiert werden, wobei ein PE-SAA-Komplex präzipitiert, der durch an sich bekannte Techniken zur Phasentrennung entfernt wird.

**11.** Verfahren zur Reinigung von Wasser von organischen Verbindungen,
**dadurch gekennzeichnet,**
daß eine Sorbens-Suspension nach einem oder mehreren der vorhergehenden Ansprüche in Wasser,

welches die organischen Fremdmoleküle enthält, hergestellt wird, diese Suspension für eine bestimmte Zeitdauer gerührt und anschließend die flüssige Phase von der festen Phase durch Präzipitation und/oder Filtration und/oder Säulentrennung entfernt wird.

**12.** Verfahren zur Reinigung von Wasser von organischen Verbindungen,
dadurch gekennzeichnet,
daß der Polyelektrolytkomplex nach einem der vorhergehenden Ansprüche in einer Säure enthalten ist und die Reinigung des Wassers von Fremdmolekülen über eine Säule durchgeführt wird.

**13.** Verfahren zur Reinigung von Wasser von organischen Verbindungen,
dadurch gekennzeichnet,
daß das Verhältnis Polyelektrolytkomplex : Wasser im Bereich von 1 : 10 - 1 : 100 liegt.

**14.** Verwendung des Komplexes nach einem oder mehreren der vorhergehenden Ansprüche als Sorbens zur Reinigung von Wasser, insbesondere von Abwässern, von organischen Verbindungen.